(19) Europäisches Patentamt
European Patent Office
Office européen des brevets

(11) EP 0 748 624 B1

(12) **FASCICULE DE BREVET EUROPEEN**

(45) Date de publication et mention de la délivrance du brevet: **08.04.1998 Bulletin 1998/15**

(51) Int. Cl.$^6$: **A61K 7/42**

(21) Numéro de dépôt: **96401103.5**

(22) Date de dépôt: **21.05.1996**

(54) **Compositions contenant un dérivé du dibenzoylméthane et un nanopigment d'oxyde de titane et utilisations**

Dibenzoylmethanderivate und Nanopigment aus Titandioxid-enthaltende Zusammensetzung und dessen Verwendungen

Composition containing a dibenzoylmethane derivative and a nanopigment of titanium dioxide and uses thereof

(84) Etats contractants désignés:
**AT BE CH DE ES FR GB IT LI NL SE**

(30) Priorité: **16.06.1995 FR 9507246**

(43) Date de publication de la demande:
**18.12.1996 Bulletin 1996/51**

(73) Titulaire: **L'OREAL**
**75008 Paris (FR)**

(72) Inventeurs:
- **Hansenne, Isabelle**
  **75017 Paris (FR)**
- **van Leeuwen, Victoria**
  **75020 Paris (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 303 995**
**WO-A-94/04131**
**GB-A- 2 184 356**

EP 0 748 624 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre un filtre particulier choisi parmi les dérivés du dibenzoylméthane avec au moins un nanopigment minéral d'oxyde de titane traité par des silanes et/ou des silicones.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreux filtres organiques solaires capables d'absorber plus ou moins sélectivement les rayons UV-A nocifs ont été proposés à ce jour dans le domaine de la cosmétique.

A cet égard, une famille de filtres UV-A particulièrement intéressante est actuellement constituée par les dérivés du dibenzoylméthane, et notamment le 4,4'-méthoxy-t.-butyldibenzoylméthane, qui présentent en effet un fort pouvoir d'adsorption intrinsèque. Ces dérivés du dibenzoylméthane, qui sont maintenant des produits bien connus en soi à titre de filtres actifs dans l'UV-A, sont notamment décrits dans les demandes de brevets français FR-A- 2 326 405 et FR-A-2 440 933, ainsi que dans la demande de brevet européen EP-A- 0 114 607 ; le 4,4'-méthoxy-t.-butyldibenzoylméthane est par ailleurs actuellement proposé à la vente sous la dénomination commerciale de "PARSOL® 1789" par la Société Givaudan.

Afin d'obtenir une protection totale sur l'ensemble du spectre solaire dans le domaine des UV, on peut associer à ces dérivés du dibenzoylméthane un filtre UV-B.

En outre, il est connu que l'addition d'un pigment minéral, et notamment d'un pigment d'oxyde de titane ($TiO_2$) permet d'augmenter les propriétés photoprotectrices des compositions solaires contenant des filtres UV.

Par conséquent, l'association entre des dérivés du dibenzoylméthane et des (nano)pigments de $TiO_2$ est très recherchée dans le domaine des compositions antisolaires.

Toutefois, il s'avère que les associations des dérivés du dibenzoylméthane avec des (nano)pigments minéraux, et plus particulièrement encore l'association 4,4'-méthoxy-t.-butyldibenzoylméthane-$TiO_2$, présentent un inconvénient qui affecte non seulement la nature et donc la qualité des produits les contenant mais également leur attrait auprès des consommateurs : en effet, on observe pour les compositions contenant ce type d'association un changement de couleur se traduisant par un jaunissement plus ou moins intense des formules. Outre le fait que ce phénomène diminue le pouvoir photoprotecteur des dérivés du dibenzoylméthane, et en particulier du 4,4'-méthoxy-t.-butyldibenzoylméthane, ce jaunissement n'est évidemment pas souhaitable d'un point de vue cosmétique.

On observe en outre que ce phénomène est particulièrement accentué dans le cas des nanopigments de $TiO_2$.

Pour résoudre ce problème, certaines solutions ont déjà été proposées dans l'art antérieur : ainsi, dans la demande de brevet japonaise JP61-215314, on a préconisé l'utilisation de séquestrants choisis parmi les acides édétiques, métaphosphoriques et polyphosphoriques et/ou leurs sels pour diminuer ce phénomène de jaunissement. Cette solution ne donne toutefois pas complètement satisfaction.

Or, à la suite d'importantes recherches menées dans le domaine de la photoprotection évoqué ci-dessus, la Demanderesse a maintenant découvert que l'utilisation de (nano)pigments d'oxyde de titane traités avec une silicone (dérivé silane ou siloxane), réduisait significativement le jaunissement habituellement constaté dans les compositions antisolaires contenant des associations classiques du type dérivé du dibenzoylméthane-pigment de $TiO_2$.

Cette découverte est à la base de la présente invention.

On notera ici qu'il est connu du document WO-A-94 04131 des compositions filtrantes photostables comprenant, dans des proportions bien définies, un dérivé de dibenzoylméthane en association avec un dérivé de benzylidène camphre. Selon ce document, le benzylidène camphre, dans les proportions indiquées, permet de photostabiliser le dérivé de dibenzoylméthane, c'est-à-dire de limiter la dégradation de ce dernier sous l'action des rayons UV, en particulier UV-A. Il est indiqué dans ce même document que ces compositions photostables peuvent en outre contenir un pigment minéral bloqueur d'UV, notamment un pigment d'oxyde de titane, qui peut être enrobé avec un composé, notamment siliconé. Toutefois, ce document ne décrit pas, ni n'enseigne, qu'en utilisant un pigment d'oxyde de titane traité avec une silicone, on peut obtenir une réduction de l'effet de jaunissement comme visée par la présente invention.

Ainsi, conformément à l'un des objets de la présente invention, il est maintenant proposé de nouvelles compositions cosmétiques, en particulier antisolaires, du type comprenant dans un support cosmétiquement acceptable, au moins un dérivé du dibenzoylméthane et au moins un pigment d'oxyde de titane, et qui sont essentiellement caractérisées par le fait que ledit pigment d'oxyde de titane est enrobé et/ou traité avec une silicone, ces compositions étant par ailleurs exemptes de composés de type benzylidène camphre de formule générale suivante :

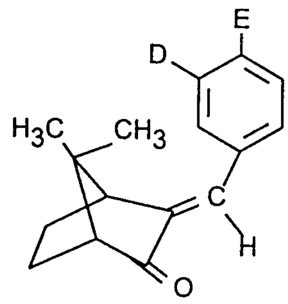

dans laquelle D et E sont des radicaux choisis indépendamment dans le groupe constitué par l'hydrogène, les radicaux alkyles en C1-C20 linéaires ou ramifiés et le radical OR dans lequel R est l'hydrogène ou un radical alkyle en C1-C20 linéaire ou ramifié.

Comme indiqué précédemment, les compositions conformes à l'invention présentent l'intérêt d'être très faiblement sujettes au phénomène de jaunissement habituellement provoqué par l'association entre un dérivé du dibenzoylméthane et des pigments $TiO_2$.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

La présente invention a encore pour objet l'utilisation d'au moins un (nano)pigment d'oxyde de titane enrobé et/ou traité avec une silicone dans des compositions antisolaires contenant un dérivé de dibenzoylméthane, dans le but d'améliorer simultanément le pouvoir photoprotecteur et la résistance au jaunissement desdites compositions.

D'autres caractéristiques, aspects et avantages de la présente invention apparaîtront à la lecture de la description détaillée qui va suivre.

Comme indiqué précédemment, les dérivés du dibenzoylméthane utilisés dans le cadre de la présente invention sont des produits déjà bien connus en soi et décrits notamment dans les documents FR-A- 2 326 405, FR-A- 2 440 933 et EP-A- 0 114 607 précités.

En particulier, les dérivés du dibenzoylméthane utilisables selon la présente invention peuvent être choisis parmi ceux répondant à la formule suivante :

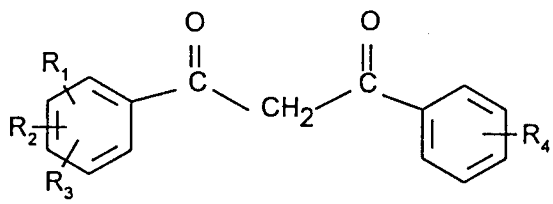

dans laquelle $R_1$, $R_2$, $R_3$ et $R_4$, identiques ou différents, représentent l'hydrogène ou un radical alkyle linéaire ou ramifié en C1-C8 ou un radical alcoxy linéaire ou ramifié en C1-C8.

Selon la présente invention, on peut bien entendu mettre en oeuvre un ou plusieurs dérivés du dibenzoylméthane.

Parmi les dérivés du dibenzoylméthane plus particulièrement visés par la présente invention, on peut notamment citer, de manière non limitative :

- le 2-méthyldibenzoylméthane,
- le 4-méthyldibenzoylméthane,

- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-méthoxy-t.-butyldibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

Parmi les dérivés du dibenzoylméthane mentionnés ci-dessus, on préfère tout particulièrement, selon la présente invention, mettre en oeuvre le 4,4'-méthoxy-t.-butyldibenzoylméthane, notamment celui proposé à la vente sous la dénomination commerciale de "PARSOL® 1789" par la Société Givaudan, ce filtre répondant donc à la formule développée suivante :

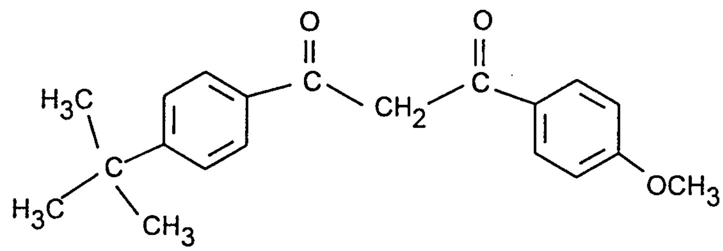

Un autre dérivé du dibenzoylméthane préféré selon la présente invention est le 4-isopropyl-dibenzoylméthane, filtre vendu sous la dénomination de "EUSOLEX® 8020" par la Société MERCK, et répondant à la formule développée suivante :

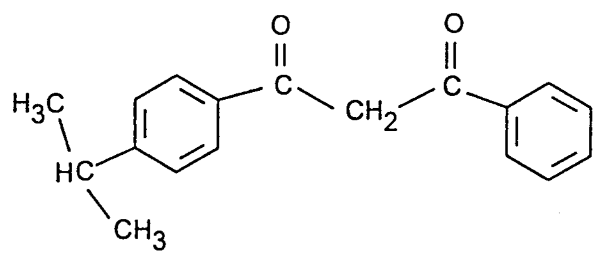

Le ou les dérivés du dibenzoylméthane peuvent être présents dans les compositions conformes à l'invention à des teneurs qui sont généralement comprises entre 0,1 % et 10 % en poids, et de de préférence à des teneurs comprises entre 0,3 % et 5 % en poids, par rapport au poids total de la composition.

Les compositions conformes à l'invention contiennent en outre des pigments minéraux d'oxyde de titane. De tels pigments comprennent également les nanopigments.

On entend par nanopigment un pigment dont la taille moyenne des particules élémentaires est choisie de 5 à 100 nm. Selon un mode préféré de réalisation de l'invention, cette taille est inférieure à 50 nm.

L'oxyde de titane peut se présenter sous forme rutile, anatase ou amorphe, mais de préférence sous forme rutile et/ou anatase.

Selon une caractéristique essentielle de la présente l'invention, les (nano)pigments qui doivent être utilisés sont des (nano)pigments d'oxydes de titane traités avec une silicone.

De façon connue, les silicones sont des polymères ou oligomères organosiliciés à structure linéaire ou cyclique, ramifiée ou réticulée, de poids moléculaire variable, obtenus par polymérisation et/ou polycondensation de silanes convenablement fonctionalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium. Les radicaux hydrocarbonés les plus courants sont les radicaux alkyle et en particulier méthyle, les radicaux fluoroalkyle, les radicaux aryle et en particulier phényle, et les radicaux alcényle et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylène (ou polyéthers) et en particulier polyoxyéthylène et/ou polyoxypropylène, les radicaux hydroxyle ou hydroxyalkyle, les groupements aminés substitués ou non, les groupements amide, les radicaux acyloxy ou acyloxyalkyle, les radicaux hydroxyalcylamino ou aminoalkyle, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Au sens de la présente invention, on entend englober et couvrir sous le terme de ⟨⟨silicones⟩⟩ également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes et les polyalkylhydrogénosiloxanes. Plus préférentiellement encore, les silicones sont choisies dans le groupe contenant l'octyl triméthyl silane, les polydiméthylsiloxanes et les polyméthylhydrogénosiloxanes.

Bien entendu, les pigments de $TiO_2$, avant leur traitement par des silicones, peuvent avoir été traités par d'autres agents de surface, en particulier par de l'alumine, de la silice, des composés de l'aluminium, des composés du silicium, ou leurs mélanges.

Les pigments utilisés dans le cadre de la présente invention peuvent être préparés selon des techniques de traitements de surface de nature chimique, électronique, mécanochimique et/ou mécanique bien connues de l'homme de l'art et décrites par exemple dans Cosmetics & Toiletries, Février 1990, Vol. 105, p 53-64. On peut également utiliser des produits commerciaux.

Les nanopigments d'oxyde de titane convenant particulièrement bien à la réalisation de la présente invention sont de préférence le $TiO_2$ traité par l'octyl triméthyl silane et dont la taille moyenne des particules élémentaires est comprise entre 25 et 40 nm tel que celui vendu sous la dénomination commerciale ⟨⟨T 805⟩⟩ par la société Degussa Silices, le $TiO_2$ enrobé par l'alumine, traité par un polydiméthylsiloxane et dont la taille moyenne des particules élémentaires est de 20 nm tel que celui vendu sous la dénomination commerciale ⟨⟨UV Titan M® 262⟩⟩ par la société Kemira, le $TiO_2$ traité par un polydiméthylsiloxane et dont la taille moyenne des particules élémentaires est de 21 nm tel que celui vendu sous la dénomination commerciale ⟨⟨70250 Cardre UF $TiO_2$ SI3®⟩⟩ par la société Cardre, le $TiO_2$ anatase/rutile traité par un polyméthylhydrogénosiloxane et dont la taille moyenne des particules élémentaires est de 25 nm tel que celui vendu sous la dénomination commerciale ⟨⟨Micro Titanium dioxide USP grade hydrophobic®⟩⟩ par la société Color Techniques, le $TiO_2$ enrobé silice/alumine, traité par une silicone et dont la taille moyenne des particules élémentaires est de 15 nm tel que celui vendu sous la dénomination commerciale ⟨⟨MT-100 SAS®⟩⟩ par la société Tayca.

Les (nano)pigments peuvent être présents dans les compositions conformes à l'invention à des teneurs qui sont généralement comprises entre 0,1 % et 30 % en poids, et de de préférence à des teneurs comprises entre 0,5 % et 20 % en poids, par rapport au poids total de la composition.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les dérivés du dibenzoylméthane mentionnés ci-avant. Ces filtres complémentaires peuvent être notamment choisis parmi les dérivés cinnamiques, les dérivés salicyliques, les dérivés du camphre, autres que ceux mentionnés dans la revendication 1, les dérivés de triazine, les dérivés de la benzophénone, les dérivés de $\beta,\beta$-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les $\alpha$-hydroxyacides, les agents antimousse, les agents hydratants, les vitamines, les parfums, les conservateurs, les tensio-actifs, les charges, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone,

volatiles ou non, les isoparaffines, les poly-$\alpha$-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, et notamment de réduction du phénomène de jaunissement, attachées intrinsèquement à l'association conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces composition peuvent se présenter en particulier sous forme d'émulsions, simples ou complexes (H/E, E/H, H/E/H ou E/H/E) telles que des crèmes, des laits, des gels ou des gels crèmes, des poudres, des bâtonnets solides et éventuellement être conditionnées en aérosols et se présenter sous forme de mousses ou de sprays. De préférence, ces compositions se présentent sous la forme d'une émulsion H/E.

Les compositions cosmétiques de l'invention peuvent être utilisées comme compositions protectrices de l'épiderme humain ou des cheveux contre les rayons ultraviolets, comme compositions antisolaires ou comme produits de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

**EXEMPLE 1**:

On a réalisé des tests comparatifs afin de mettre en évidence l'amélioration apportée par l'utilisation d'un nanopigment d'oxyde de titane enrobé avec une silicone dans les associations 4,4'-méthoxy-t.-butyldibenzoylméthanenanopigment d'oxyde de titane.

Pour cela, on a réalisé dix dispersions contenant chacune, dans un support identique (benzoate d'alkyle en C12-C15), du 4,4'-méthoxy-t.-butyldibenzoylméthane en association avec un nanopigment d'oxyde de titane, en faisant varier la nature du nanopigment d'oxyde de titane : pour les dispersions 1, 2 et 3, les pigments utilisés sont des nanopigments d'oxyde de titane classiques (non enrobés) ; pour les dispersions 4 et 5, les pigments utilisés sont des nanopigments d'oxyde de titane enrobés avec des composés autres qu'une silicone ; et pour les dispersions 6, 7, 8, 9, et 10, les pigments utilisés sont des nanopigments d'oxyde de titane enrobés avec une silicone, conformément à la présente invention.

Afin de mesurer la variation de couleur induite par la présence du 4,4'-méthoxy-t.-butyldibenzoylméthane en association avec un nanopigment d'oxyde de titane dans la dispersion, on a réalisé, pour chacune de ces dispersions 1 à 10, une dispersion étalon ne contenant pas le 4,4'-mèthoxy-t.-butyldibenzoylméthane mais contenant, dans le même support, le nanopigment de la dispersion 1 à 10 que l'on veut tester.

Les différentes dispersions ont été réalisées de la manière suivante :

Dispersions 1 à 10 :

Dans un flacon, on a ajouté, à 2 g de nanopigment d'oxyde de titane, 18 g d'un mélange formé de 16 g de benzoate d'alkyle en C12-C15 vendu sous la dénomination commerciale 《Finsolv TN® 》 par Finetex et de 2 g du 4,4'-méthoxy-t.-butyldibenzoylméthane vendu sous la dénomination commerciale 《Parsol® 1789 》 par Givaudan, ce mélange étant préalablement homogénéisé à 80°C. Puis on a secoué le flacon pour homogénéiser la dispersion.

Dispersions étalons :

Elles ont été réalisées selon le même mode opératoire que pour les dispersions 1 à 10 en mettant en oeuvre 18 g de benzoate d'alkyle en C12-C15 (absence de 《Parsol® 1789 》).

Les dispersions 1 à 10 ont ensuite été conservées 24h à l'abri de la lumière. Les mesures colorimétriques ont été effectuées à l'aide d'un colorimètre Minolta CM 1000. Un échantillon de chaque dispersion réhomogénéisée a été analysé. Pour chaque échantillon, les valeurs de L*, a* et b* ( où L représente la luminance, a représente l'axe rouge-vert (-a = vert, +a = rouge) et b représente l'axe jaune-bleu (-b =bleu, +b =jaune)) ont été mesurées et la variation de couleur ΔE* a été calculée à partir des variations ΔL*, Δa* et Δb* entre la dispersion 1 à10 à tester et sa dispersion étalon selon l'équation suivante :

$$\Delta E^* = \sqrt{(\Delta L^*)^2 + (\Delta a^*)^2 + (\Delta b^*)^2}$$

Plus ΔE* est faible, plus la variation de couleur est réduite.

Les compositions des dispersions 1 à 10 et les résultats obtenus sont consignés dans le tableau (I) suivant:

Tableau (I)

| Dispersion | Nanopigment | $\Delta E^*$ |
|---|---|---|
| 1 comparatif | $TiO_2$ anatase 30 nm vendu sous la dénomination 《Transparent PW® 》 par Les colorants Wackherr | 19 |
| 2 comparatif | $TiO_2$ 35 nm vendu sous la dénomination 《MT 500 B® 》 par Tayca | 12 |
| 3 comparatif | $TiO_2$ rutile 70 nm vendu sous la dénomination 《MT-Trial® Product N° 482 》 par Tayca | 10 |
| 4 comparatif | $TiO_2$ rutile 15 nm enrobé alumine/silice vendu sous la dénomination 《MT 100 SA® 》 par Tayca | 12 |
| 5 comparatif | $TiO_2$ rutile 15 nm enrobé hydroxyde d'alumine/acide stéarique vendu sous la dénomination 《MT 100 T® 》 par Tayca | 9 |
| 6 invention | $TiO_2$ 25-40 nm traité octyl triméthyl silane vendu sous la dénomination 《T 805® 》 par Degussa | 6 |
| 7 invention | $TiO_2$ 20 nm enrobé alumine traité polydiméthylsiloxane vendu sous la dénomination 《UV Titan M 262® 》 par Kemira | 4 |
| 8 invention | $TiO_2$ 21 nm traité polydiméthylsiloxane vendu sous la dénomination 《70250 Cardre UF $TiO_2$ SI3® 》 par Cardre | 4 |
| 9 invention | $TiO_2$ anatase/rutile 25 nm traité polyméthylhydrogénosiloxane vendu sous la dénomination 《Micro Titanium dioxide USP grade hydrophobic® 》 par Color Techniques | 3 |
| 10 invention | $TiO_2$ 15 nm enrobé silice/alumine traité silicone vendu sous la dénomination 《MT 100 SAS® 》 par Tayca | 3 |

Ces résultats montrent clairement que l'enrobage du nanopigment d'oxyde de titane par une silicone, que ce nanopigment soit déjà enrobé par d'autres composés chimiques (cas des dispersions 7 et 10) ou non (dispersions 6, 8 et 9), diminue significativement le changement de couleur, et en particulier le jaunissement, dû à l'association 4,4'-méthoxy-t.-butyldibenzoylméthanenanopigment d'oxyde de titane par rapport aux dispersions contenant des nanopigments d'oxyde de titane non enrobés (dispersions 1, 2 et 3) ou des nanopigments d'oxydes de titane dont l'enrobage ne contient pas de silicone (dispersions 4 et 5).

**EXEMPLE 2**:

La composition suivante donne un exemple concret d'une formule solaire haute protection conforme à l'invention sous la forme d'une émulsion H/E. Les quantités sont exprimées en % de poids par rapport au poids total de la composition :

Phase A:

| | |
|---|---|
| - 4,4'-méthoxy-t.-butyldibenzoylméthane vendu sous la dénomination commerciale ⟨⟨Parsol® 1789 ⟩⟩ par Givaudan | 3 % |
| - benzoate d'alkyle en C12-C15 vendu sous la dénomination commerciale ⟨⟨Finsolv TN® ⟩⟩ par Finetex | 2 % |
| - alcool cétylique vendu sous la dénomination commerciale ⟨⟨Lanette 16 NF® ⟩⟩ par Henkel | 1,5 % |
| - diméthicone vendue sous la dénomination commerciale ⟨⟨DC 200 Fluid® ⟩⟩ par Dow Corning | 0,5 % |
| - octocrylène vendu sous la dénomination commerciale ⟨⟨Uvinul N 539® ⟩⟩ par BASF | 10 % |
| - mélange de PEG-100 stéarate et de glycéryl stéarate vendu sous la dénomination commerciale ⟨⟨Arlacel 165® ⟩⟩ par ICI | 1,5 % |
| - $TiO_2$ traité alkyl silane vendu sous la dénomination commerciale ⟨⟨T 805® ⟩⟩ par Degussa Silices | 5 % |

Phase B:

| | |
|---|---|
| - glycérol | 7 % |
| - carbomer vendu sous la dénomination commerciale ⟨⟨Carbopol 980 ⟩⟩ par Goodrich | 0,2 % |
| - disodium EDTA vendu sous la dénomination commerciale ⟨⟨Edeta BD® ⟩⟩ par Basf | 0,1 % |
| - eau | qsp 100 % |

Phase C:

| | |
|---|---|
| - cyclométhicone vendue sous la dénomination commerciale ⟨⟨DC 245 Fluid® ⟩⟩ par Dow Corning | 10 % |
| Conservateurs | qs |
| Neutralisant | qs pH = 7 |

Mode opératoire : l'émulsion ci-dessus a été réalisée de la manière suivante : on a porté la phase A à 80°C pour fondre l'ensemble des constituants. On a également porté la phase B à 80°C après y avoir préalablement dispersé le carbomer. Puis on a versé la phase A sur la phase B à chaud vers 65°C. On a agité le mélange 10 minutes pour émulsification. On a ensuite ajouté la phase C sous agitation puis on a neutralisé le carbomer. Enfin, on a terminé l'émulsion par agitation jusqu'à refroidissement complet (≈ 20-25°C).

## Revendications

1. Composition cosmétique à usage topique, en particulier pour la photoprotection de la peau et/ou des cheveux, du type comprenant dans un support cosmétiquement acceptable, au moins un dérivé du dibenzoylméthane et au moins un pigment d'oxyde de titane, et qui sont essentiellement caractérisées par le fait que ledit pigment d'oxyde de titane est enrobé et/ou traité avec une silicone, cette composition étant par ailleurs exempte de composés de type benzylidène camphre de formule générale suivante :

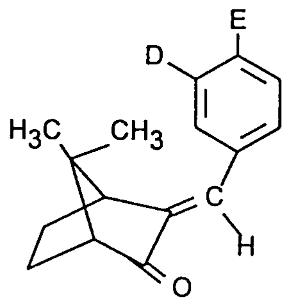

dans laquelle D et E sont des radicaux choisis indépendamment dans le groupe constitué par l'hydrogène, les radicaux alkyles en C1-C20 linéaires ou ramifiés et le radical OR dans lequel R est l'hydrogène ou un radical alkyle en C1-C20 linéaire ou ramifié.

2. Composition selon la revendication 1, caractérisée par le fait que le dérivé du dibenzoylméthane est choisi parmi ceux répondant à la formule suivante :

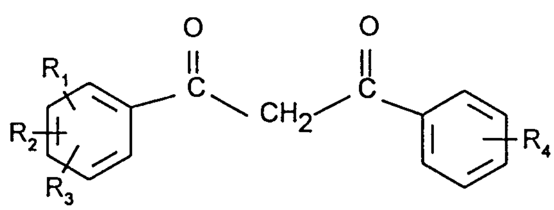

dans laquelle $R_1$, $R_2$, $R_3$ et $R_4$, identiques ou différents, représentent l'hydrogène ou un radical alkyle linéaire ou ramifié en C1-C8 ou un radical alcoxy linéaire ou ramifié en C1-C8.

3. Composition selon la revendication 2, caractérisée par le fait que le dérivé du dibenzoylméthane est choisi dans le groupe comprenant le 2-méthyldibenzoylméthane, le 4-méthyldibenzoylméthane, le 4-isopropyldibenzoylméthane, le 4-tert.-butyldibenzoylméthane, le 2,4-diméthyldibenzoylméthane, le 2,5-diméthyldibenzoylméthane, le 4,4'-diisopropyldibenzoylméthane, le 4,4'-méthoxy-t.-butyldibenzoylméthane, le 2-méthyl-5-isopropyl-4'-méthoxydibenzoylméthane, le 2-méthyl-5-tert.-butyl-4'-méthoxydibenzoylméthane, le 2,4-diméthyl-4'-méthoxydibenzoylméthane, le 2,6-diméthyl4-tert.-butyl-4'-méthoxydibenzoylméthane.

4. Composition selon la revendication 3, caractérisée par le fait que le dérivé du dibenzoylméthane est le 4,4'-méthoxy-t.-butyldibenzoylméthane.

5. Composition selon la revendication 3, caractérisée par le fait que le dérivé du dibenzoylméthane est le 4-isopropyldibenzoylméthane.

6. Composition selon l'une quelconque des revendications précédentes, caractérisée par le fait que ledit pigment est, avant traitement par une silicone, enrobé par un agent d'enrobage autre qu'une silicone.

7. Composition selon la revendication 6, caractérisée par le fait que l'agent d'enrobage est choisi parmi l'alumine, la silice, les composés de l'aluminium, les composés du silicium, ou leurs mélanges.

8. Composition selon l'une quelconque des revendications précédentes, caractérisée par le fait que la taille moyenne des particules élémentaires dudit pigment est comprise entre 5 et 100 nm.

9. Composition selon la revendication 8, caractérisée par le fait que ladite taille est inférieure à 50 nm.

10. Composition selon l'une quelconque des revendications précédentes, caractérisée par le fait que la silicone est choisie dans le groupe contenant les alkyl silanes, les polydialkylsiloxanes et les polyalkylhydrogénosiloxanes.

11. Composition selon la revendication 10 caractérisée par le fait que la silicone est choisie dans le groupe contenant l'octyl triméthyl silane, les polydiméthylsiloxanes et les polyméthylhydrogénosiloxanes.

12. Composition selon l'une quelconque des revendications 1 à 11, caractérisée par le fait que le pigment est un oxyde de titane traité par l'octyl triméthyl silane.

13. Composition selon l'une quelconque des revendications 1 à 11, caractérisée par le fait que le pigment est un oxyde de titane enrobé par l'alumine, traité par un polydiméthylsiloxane.

14. Composition selon l'une quelconque des revendications 1 à 11, caractérisée par le fait que le pigment est un oxyde de titane traité par un polydiméthylsiloxane.

15. Composition selon l'une quelconque des revendications 1 à 11, caractérisée par le fait que le pigment est un oxyde de titane anatase/rutile traité par un polyméthylhydrogénosiloxane.

16. Composition selon l'une quelconque des revendications 1 à 11, caractérisée par le fait que le pigment est un oxyde de titane enrobé silice/alumine, traité par une silicone.

17. Composition selon l'une quelconque des revendications précédentes, caractérisée par le fait que le dérivé du dibenzoylméthane est présent dans la composition dans une proportion comprise entre 0,1 et 10 % en poids, par rapport au poids total de la composition.

18. Composition selon la revendication 17, caractérisée par le fait que le dérivé du dibenzoylméthane est présent dans la composition dans une proportion comprise entre 0,3 et 5 % en poids, par rapport au poids total de la composition.

19. Composition selon l'une quelconque des revendications précédentes, caractérisée par le fait que le pigment est présent dans la composition dans une proportion comprise entre 0,1 et 30 % en poids, par rapport au poids total de la composition.

20. Composition selon la revendication 19, caractérisée par le fait que le pigment est présent dans la composition dans une proportion comprise entre 0,5 et 20 % en poids, par rapport au poids total de la composition.

21. Utilisation d'une composition telle que définie à l'une des revendications 1 à 20 comme, ou pour la fabrication de, compositions cosmétiques destinées à la protection de la peau et/ou des cheveux contre le rayonnement ultraviolet, en particulier le rayonnement solaire.

22. Procédé de traitement cosmétique pour la protection de la peau et/ou des cheveux contre le rayonnement ultraviolet, en particulier le rayonnement solaire, consistant à appliquer sur ces derniers une quantité efficace d'une composition telle que définie à l'une quelconque des revendications 1 à 20.

23. Utilisation d'au moins un pigment d'oxyde de titane enrobé et/ou traité avec une silicone dans des compositions antisolaires contenant un dérivé de dibenzoylméthane, dans le but d'améliorer simultanément le pouvoir photoprotecteur et la résistance au jaunissement desdites compositions.

24. Utilisation selon la revendication 23, caractérisée par le fait que le dérivé du dibenzoylméthane est choisi parmi ceux répondant à la formule suivante :

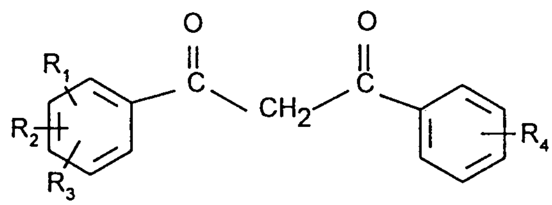

dans laquelle $R_1$, $R_2$, $R_3$ et $R_4$, identiques ou différents, représentent l'hydrogène ou un radical alkyle linéaire ou ramifié en C1-C8 ou un radical alcoxy linéaire ou ramifié en C1-C8.

**25.** Utilisation selon la revendication 24, caractérisée par le fait que le dérivé du dibenzoylméthane est choisi dans le groupe comprenant le 2-méthyldibenzoylméthane, le 4-méthyldibenzoylméthane, le 4-isopropyldibenzoylméthane, le 4-tert.-butyldibenzoylméthane, le 2,4-diméthyldibenzoylméthane, le 2,5-diméthyldibenzoylméthane, le 4,4'-diisopropyldibenzoylméthane, le 4,4'-méthoxy-t.-butyldibenzoylméthane, le 2-méthyl-5-isopropyl-4'-méthoxydibenzoylméthane, le 2-méthyl-5-tert.-butyl-4'-méthoxydibenzoylméthane, le 2,4-diméthyl-4'-méthoxydibenzoylméthane, le 2,6-diméthyl-4-tert.-butyl-4'-méthoxydibenzoylméthane.

**26.** Utilisation selon la revendication 25, caractérisée par le fait que le dérivé du dibenzoylméthane est le 4,4'-méthoxy-t.-butyldibenzoylméthane.

**27.** Utilisation selon la revendication 25, caractérisée par le fait que le dérivé du dibenzoylméthane est le 4-isopropyldibenzoylméthane.

**28.** Utilisation selon l'une quelconque des revendications 23 à 27, caractérisée par le fait que ledit pigment est, avant traitement par une silicone, enrobé par un agent d'enrobage autre qu'une silicone.

**29.** Utilisation selon la revendication 28, caractérisée par le fait que l'agent d'enrobage est choisi parmi l'alumine, la silice, les composés de l'aluminium, les composés du silicium, ou leurs mélanges.

**30.** Utilisation selon l'une quelconque des revendications 23 à 29, caractérisée par le fait que la taille moyenne des particules élémentaires dudit pigment est comprise entre 5 et 100 nm.

**31.** Utilisation selon la revendication 30, caractérisée par le fait que ladite taille est inférieure à 50 nm.

**32.** Utilisation selon l'une quelconque des revendications 23 à 31, caractérisée par le fait que la silicone est choisie dans le groupe contenant les alkyl silanes, les polydialkylsiloxanes et les polyalkylhydrogénosiloxanes.

**33.** Utilisation selon la revendication 32 caractérisée par le fait que la silicone est choisie dans le groupe contenant l'octyl triméthyl silane, les polydiméthylsiloxanes et les polyméthylhydrogénosiloxanes.

**34.** Utilisation selon l'une quelconque des revendications 23 à 33, caractérisée par le fait que le pigment est un oxyde de titane traité par l'octyl triméthyl silane.

**35.** Utilisation selon l'une quelconque des revendications 23 à 33, caractérisée par le fait que le pigment est un oxyde de titane enrobé par l'alumine, traité par un polydiméthylsiloxane.

**36.** Utilisation selon l'une quelconque des revendications 23 à 33, caractérisée par le fait que le pigment est un oxyde de titane traité par un polydiméthylsiloxane.

**37.** Utilisation selon l'une quelconque des revendications 23 à 33, caractérisée par le fait que le pigment est un oxyde de titane anatase/rutile traité par un polyméthylhydrogénosiloxane.

**38.** Utilisation selon l'une quelconque des revendications 23 à 33, caractérisée par le fait que le pigment est un oxyde de titane enrobé silice/alumine, traité par une silicone.

**39.** Utilisation selon l'une quelconque des revendications 23 à 38, caractérisée par le fait que le dérivé du dibenzoylméthane est présent dans la composition dans une proportion comprise entre 0,1 et 10 % en poids, par rapport au poids total de la composition.

**40.** Utilisation selon la revendication 39, caractérisée par le fait que le dérivé du dibenzoylméthane est présent dans la composition dans une proportion comprise entre 0,3 et 5 % en poids, par rapport au poids total de la composition.

**41.** Utilisation selon l'une quelconque des revendications 23 à 40, caractérisée par le fait que le pigment est présent dans la composition finale dans une proportion comprise entre 0,1 et 30 % en poids, par rapport au poids total de ladite composition.

**42.** Utilisation selon la revendication 41, caractérisée par le fait que le pigment est présent dans la composition finale dans une proportion comprise entre 0,5 et 20 % en poids, par rapport au poids total de ladite composition.

## Claims

**1.** Cosmetic composition for topical use, in particular for the photoprotection of the skin and/or hair, of the type comprising, in a cosmetically acceptable vehicle, at least one dibenzoylmethane derivative and at least one titanium oxide pigment, which composition is essentially characterized in that the said titanium oxide pigment is coated and/or treated with a silicone, this composition being, moreover, devoid of compounds of the benzylidenecamphor type, of the following general formula:

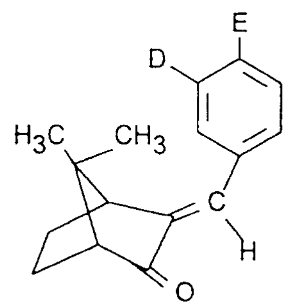

in which D and E are radicals chosen independently from the group consisting of hydrogen, linear or branched $C_1$-$C_{20}$ alkyl radicals and the radical OR in which R is hydrogen or a linear or branched $C_1$-$C_{20}$ alkyl radical.

**2.** Composition according to Claim 1, characterized in that the dibenzoylmethane derivative is chosen from those corresponding to the following formula:

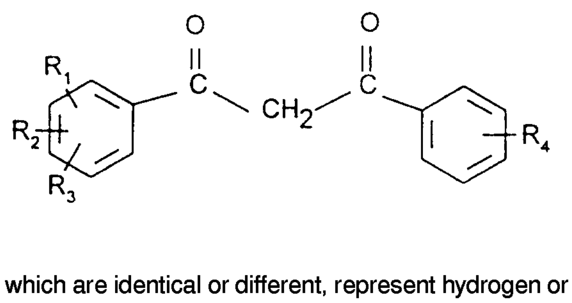

in which $R_1$, $R_2$, $R_3$ and $R_4$, which are identical or different, represent hydrogen or a linear or branched $C_1$-$C_8$ alkyl radical or a linear or branched $C_1$-$C_8$ alkoxy radical.

**3.** Composition according to Claim 2, characterized in that the dibenzoylmethane derivative is chosen from the group consisting of 2-methyldibenzoylmethane, 4-methyldibenzoylmethane, 4-isopropyldibenzoylmethane, 4-tert-butyldibenzoylmethane, 2,4-dimethyldibenzoylmethane, 2,5-dimethyldibenzoylmethane, 4,4'-diisopropyldibenzoylmethane, 4-methoxy-4'-tert-butyldibenzoylmethane, 2-methyl-5-isopropyl-4'-methoxydibenzoylmethane, 2-methyl-5-tert-butyl4'-methoxydibenzoylmethane, 2,4-dimethyl-4'-methoxydibenzoylmethane, 2,6-dimethyl-4-tert-butyl-4'-methooxydibenzoylmethane.

4. Composition according to Claim 3, characterized in that the dibenzoylmethane derivative is 4-methoxy-4'-tert-butyldibenzoylmethane.

5. Composition according to Claim 3, characterized in that the dibenzoylmethane derivative is 4-isopropyldibenzoylmethane.

6. Composition according to any one of the preceding claims, characterized in that the said pigment is, before treatment with a silicone, coated with a coating agent other than a silicone.

7. Composition according to Claim 6, characterized in that the coating agent is chosen from alumina, silica, aluminium compounds, silicon compounds, and mixtures thereof.

8. Composition according to any one of the preceding claims, characterized in that the mean size of the primary particles of the said pigment is between 5 and 100 nm.

9. Composition according to Claim 8, characterized in that the said size is less than 50 nm.

10. Composition according to any one of the preceding claims, characterized in that the silicone is chosen from the group consisting of alkylsilanes, polydialkylsiloxanes and polyalkylhydridosiloxanes.

11. Composition according to Claim 10, characterized in that the silicone is chosen from the group consisting of octyltrimethylsilane, polydimethylsiloxanes and polymethylhydridosiloxanes.

12. Composition according to any one of Claims 1 to 11, characterized in that the pigment is a titanium oxide treated with octyltrimethylsilane.

13. Composition according to any one of Claims 1 to 11, characterized in that the pigment is a titanium oxide coated with alumina, treated with a polydimethylsiloxane.

14. Composition according to any one of Claims 1 to 11, characterized in that the pigment is a titanium oxide treated with a polydimethylsiloxane.

15. Composition according to any one of Claims 1 to 11, characterized in that the pigment is an anatase/rutile titanium oxide treated with a polymethylhydridosiloxane.

16. Composition according to any one of Claims 1 to 11, characterized in that the pigment is a titanium oxide coated with silica/alumina, treated with a silicone.

17. Composition according to any one of the preceding claims, characterized in that the dibenzoylmethane derivative is present in the composition in a proportion of between 0.1 and 10 % by weight relative to the total weight of the composition.

18. Composition according to Claim 17, characterized in that the dibenzoylmethane derivative is present in the composition in a proportion of between 0.3 and 5 % by weight relative to the total weight of the composition.

19. Composition according to any one of the preceding claims, characterized in that the pigment is present in the composition in a proportion of between 0.1 and 30 % by weight relative to the total weight of the composition.

20. Composition according to Claim 19, characterized in that the pigment is present in the composition in a proportion of between 0.5 and 20 % by weight relative to the total weight of the composition.

21. Use of a composition as defined in one of Claims 1 to 20 as, or for the production of, cosmetic compositions which are intended for the protection of skin and/or hair against ultraviolet radiation, especially solar radiation.

22. Method of cosmetic treatment for the protection of skin and/or hair against ultraviolet radiation, especially solar radiation, which consists in applying to the skin and/or hair an effective quantity of a composition as defined in any one of Claims 1 to 20.

**23.** Use of at least one titanium oxide pigment coated and/or treated with a silicone in antisun compositions containing a dibenzoylmethane derivative, in the aim of improving simultaneously the photoprotective power and the resistance to yellowing of the said compositions.

**24.** Use according to Claim 23, characterized in that the dibenzoylmethane derivative is chosen from those corresponding to the following formula:

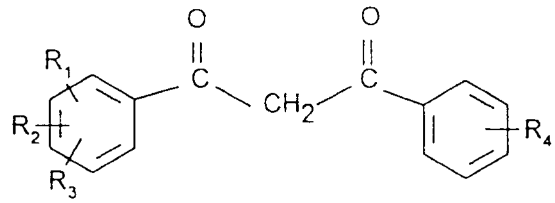

in which $R_1$, $R_2$, $R_3$ and $R_4$, which are identical or different, represent hydrogen or a linear or branched $C_1$-$C_8$ alkyl radical or a linear or branched $C_1$-$C_8$ alkoxy radical.

**25.** Use according to Claim 24, characterized in that the dibenzoylmethane derivative is chosen from the group consisting of 2-methyldibenzoylmethane, 4-methyldibenzoylmethane, 4-isopropyldibenzoylmethane, 4-tert-butyldibenzoylmethane, 2,4-dimethyldibenzoylmethane, 2,5-dimethyldibenzoylmethane, 4,4'-diisopropyldibenzoylmethane, 4-methoxy-4'-tert-butyldibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane, 2,6-dimethyl-4-tert-butyl-4'-methoxydibenzoylmethane.

**26.** Use according to Claim 25, characterized in that the dibenzoylmethane derivative is 4-methoxy-4'-tert-butyldibenzoylmethane.

**27.** Use according to Claim 25, characterized in that the dibenzoylmethane derivative is 4-isopropyldibenzoylmethane.

**28.** Use according to any one of Claims 23 to 27, characterized in that the said pigment is, before treatment with a silicone, coated with a coating agent other than a silicone.

**29.** Use according to Claim 28, characterized in that the coating agent is chosen from alumina, silica, aluminium compounds, silicon compounds, and mixtures thereof.

**30.** Use according to any one of Claims 23 to 29, characterized in that the mean size of the primary particles of the said pigment is between 5 and 100 nm.

**31.** Use according to Claim 30, characterized in that the said size is less than 50 nm.

**32.** Use according to any one of Claims 23 to 31, characterized in that the silicone is chosen from the group consisting of alkylsilanes, polydialkylsiloxanes and polyalkylhydridosiloxanes.

**33.** Use according to Claim 32, characterized in that the silicone is chosen from the group consisting of octyltrimethylsilane, polydimethylsiloxanes and polymethylhydridosiloxanes.

**34.** Use according to any one of Claims 23 to 33, characterized in that the pigment is a titanium oxide treated with octyltrimethylsilane.

**35.** Use according to any one of Claims 23 to 33, characterized in that the pigment is a titanium oxide coated with alumina, treated with a polydimethylsiloxane.

**36.** Use according to any one of Claims 23 to 33, characterized in that the pigment is a titanium oxide treated with a polydimethylsiloxane.

**37.** Use according to any one of Claims 23 to 33, characterized in that the pigment is an anatase/rutile titanium oxide

treated with a polymethylhydridosiloxane.

**38.** Use according to any one of Claims 23 to 33, characterized in that the pigment is a titanium oxide coated with silica/alumina, treated with a silicone.

**39.** Use according to any one of Claims 23 to 38, characterized in that the dibenzoylmethane derivative is present in the composition in a proportion of between 0.1 and 10 % by weight relative to the total weight of the composition.

**40.** Use according to Claim 39, characterized in that the dibenzoylmethane derivative is present in the composition in a proportion of between 0.3 and 5 % by weight relative to the total weight of the composition.

**41.** Use according to any one of Claims 23 to 40, characterized in that the pigment is present in the final composition in a proportion of between 0.1 and 30 % by weight relative to the total weight of the said composition.

**42.** Use according to Claim 41, characterized in that the pigment is present in the final composition in a proportion of between 0.5 and 20 % by weight relative to the total weight of the said composition.

**Patentansprüche**

**1.** Kosmetische Zusammensetzungen zur topischen Anwendung, insbesondere zum Schutz der Haut und/oder der Haare gegen Licht, des Typs, der in einem kosmetisch akzeptablen Träger mindestens ein Dibenzoylmethanderivat und mindestens ein Pigment von Titandioxid enthält, die im wesentlichen dadurch gekennzeichnet sind, daß das Pigment von Titandioxid mit einem Silicon umhüllt und/oder behandelt ist, wobei diese Zusammensetzungen im übrigen keine Verbindungen vom Typ eines Benzylidencamphers der folgenden allgemeinen Formel enthalten:

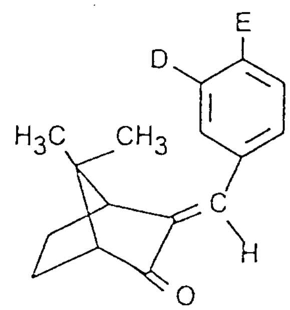

worin D und E Gruppen sind, die unabhängig voneinander unter Wasserstoff, geradkettigen oder verzweigten $C_{1-20}$-Alkylgruppen und der Gruppe OR, in der R Wasserstoff oder eine geradkettige oder verzweigte $C_{1-20}$-Alkylgruppe bedeutet, ausgewählt sind.

**2.** Zusammensetzungen nach Anspruch 1, dadurch gekennzeichnet, daß das Dibenzoylmethanderivat unter den Verbindungen der folgenden Formel ausgewählt ist:

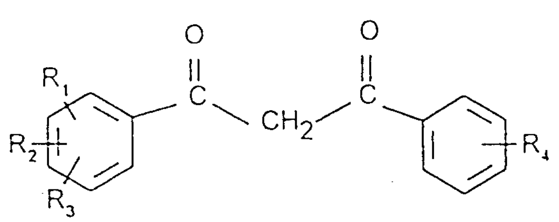

worin $R_1$, $R_2$, $R_3$ und $R_4$, die identisch oder voneinander verschieden sind, Wasserstoff oder eine geradkettige oder verzweigte $C_{1-8}$-Alkylgruppe oder eine geradkettige oder verzweigte $C_{1-8}$-Alkoxygruppe bedeuten.

**3.** Zusammensetzungen nach Anspruch 2, dadurch gekennzeichnet, daß das Dibenzoylmethanderivat unter 2-Methyldibenzoylmethan, 4-Methyldibenzoylmethan, 4-Isopropyldibenzoylmethan, 4-*tert*.-Butyldibenzoylmethan, 2,4-Dimethyldibenzoylmethan, 2,5-Dimethyldibenzoylmethan, 4,4'-Diisopropyldibenzoylmethan, 4,4'-Methoxy-*tert*.-butyldibenzoylmethan, 2-Methyl-5-isopropyl-4'-methoxydibenzoylmethan, 2-Methyl-5-*tert*.-butyl-4'-methoxydibenzoylmethan, 2,4-Dimethyl-4'-methoxydibenzoylmethan und 2,6-Dimethyl-4-*tert*.-butyl-4'-methoxydibenzoylmethan ausgewählt ist.

**4.** Zusammensetzungen nach Anspruch 3, dadurch gekennzeichnet, daß das Dibenzoylmethanderivat das 4,4'-Methoxy-*tert*.-butyldibenzoylmethan ist.

**5.** Zusammensetzungen nach Anspruch 3, dadurch gekennzeichnet, daß das Dibenzoylmethanderivat das 4-Isopropyldibenzoylmethanderivat ist.

**6.** Zusammensetzungen nach einem der vorhergehenden Ansprüche, dadurch gekennzeichnet, daß das Pigment vor der Behandlung mit einem Silicon mit einem Hüllstoff, der von einem Silicon verschieden ist, umhüllt ist.

**7.** Zusammensetzungen nach Anspruch 6, dadurch gekennzeichnet, daß der Hüllstoff unter Aluminiumoxid, Siliciumdioxid, Aluminiumverbindungen und Siliciumverbindungen oder den Gemischen dieser Verbindungen ausgewählt ist.

**8.** Zusammensetzungen nach einem der vorhergehenden Ansprüche, dadurch gekennzeichnet, daß die mittlere Größe der Elementarteilchen des Pigments im Bereich von 5 bis 100 nm liegt.

**9.** Zusammensetzungen nach Anspruch 8, dadurch gekennzeichnet, daß die Größe unter 50 nm liegt.

**10.** Zusammensetzungen nach einem der vorhergehenden Ansprüche, dadurch gekennzeichnet, daß das Silicon unter den Alkylsilanen, den Polydialkylsiloxanen und den Polyalkylhydrogenosiloxanen ausgewählt ist.

**11.** Zusammensetzungen nach Anspruch 10, dadurch gekennzeichnet, daß das Silicon unter Octyltrimethylsilan, den Polydimethylsiloxanen und den Polymethylhydrogenosiloxanen ausgewählt ist.

**12.** Zusammensetzungen nach einem der Ansprüche 1 bis 11, dadurch gekennzeichnet, daß das Pigment ein mit Octyltrimethylsilan behandeltes Titandioxid ist.

**13.** Zusammensetzungen nach einem der Ansprüche 1 bis 11, dadurch gekennzeichnet, daß das Pigment ein mit Aluminiumoxid umhülltes und mit einem Polydimethylsiloxan behandeltes Titandioxid ist.

**14.** Zusammensetzungen nach einem der Ansprüche 1 bis 11, dadurch gekennzeichnet, daß das Pigment ein mit einem Polydimethylsiloxan behandeltes Titandioxid ist.

**15.** Zusammensetzungen nach einem der Ansprüche 1 bis 11, dadurch gekennzeichnet, daß das Pigment ein mit einem Polymethylhydrogenosiloxan behandeltes Anatas/Rutil-Titandioxid ist.

**16.** Zusammensetzungen nach einem der Ansprüche 1 bis 11, dadurch gekennzeichnet, daß das Pigment ein mit Siliciumdioxid/Aluminiumoxid umhülltes und mit einem Silicon behandeltes Titandioxid ist.

**17.** Zusammensetzungen nach einem der vorhergehenden Ansprüche, dadurch gekennzeichnet, daß das Dibenzoylmethanderivat in der Zusammensetzung in einem Mengenanteil im Bereich von 0,1 bis 10 Gew.-%, bezogen auf das Gesamtgewicht der Zusammensetzung, vorliegt.

**18.** Zusammensetzungen nach Anspruch 17, dadurch gekennzeichnet, daß das Dibenzoylmethanderivat in der Zusammensetzung in einem Mengenanteil im Bereich von 0,3 bis 5 Gew.-%, bezogen auf das Gesamtgewicht der Zusammensetzung, vorliegt.

**19.** Zusammensetzungen nach einem der vorhergehenden Ansprüche, dadurch gekennzeichnet, daß das Pigment in der Zusammensetzung in einem Mengenanteil im Bereich von 0,1 bis 30 Gew.-%, bezogen auf das Gesamtgewicht der Zusammensetzung, vorliegt.

**20.** Zusammensetzungen nach Anspruch 19, dadurch gekennzeichnet, daß das Pigment in der Zusammensetzung in

einem Mengenanteil im Bereich von 0,5 bis 20 Gew.-%, bezogen auf das Gesamtgewicht der Zusammensetzung, vorliegt.

**21.** Verwendung einer Zusammensetzung nach einem der Ansprüche 1 bis 20 als kosmetische Zusammensetzung, die zum Schutz der Haut und/oder der Haare gegen UV-Strahlung und insbesondere gegen Sonnenlicht bestimmt ist, oder zu deren Herstellung.

**22.** Kosmetisches Behandlungsverfahren zum Schutz der Haut und/oder der Haare gegen UV-Strahlung und insbesondere gegen Sonnenlicht, das darin besteht, auf die Haut und/oder die Haare eine wirksame Menge einer Zusammensetzung nach Anspruch 1 bis 20 aufzutragen.

**23.** Verwendung mindestens eines mit einem Silicon umhüllten und/oder behandelten Pigments von Titandioxid in Sonnenschutzmitteln, die ein Dibenzoylmethanderivat enthalten, zur gleichzeitigen Verbesserung das Schutzvermögen dieser Zusammensetzungen gegen Licht und ihrer Beständigkeit gegenüber Gelbfärbung.

**24.** Verwendung nach Anspruch 23, dadurch gekennzeichnet, daß das Dibenzoylmethanderivat unter den Verbindungen der folgenden Formel ausgewählt ist:

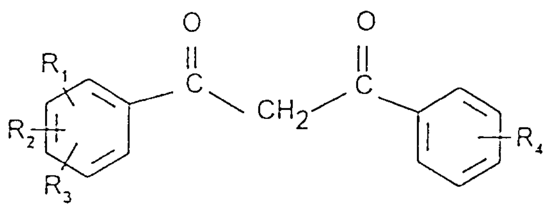

worin $R_1$, $R_2$, $R_3$ und $R_4$, die identisch oder voneinander verschieden sind, Wasserstoff oder eine geradkettige oder verzweigte $C_{1-8}$-Alkylgruppe oder eine geradkettige oder verzweigte $C_{1-8}$-Alkoxygruppe bedeuten.

**25.** Verwendung nach Anspruch 24, dadurch gekennzeichnet, daß das Dibenzoylmethanderivat unter 2-Methyldibenzoylmethan, 4-Methyldibenzoylmethan, 4-Isopropyldibenzoylmethan, 4-*tert.*-Butyldibenzoylmethan, 2,4-Dimethyldibenzoylmethan, 2,5-Dimethyldibenzoylmethan, 4,4'-Diisopropyldibenzoylmethan, 4,4'-Methoxy-*tert.*-butyldibenzoylmethan, 2-Methyl-5-isopropyl-4'-methoxydibenzoylmethan, 2-Methyl-5-*tert.*-butyl-4'-methoxydibenzoylmethan, 2,4-Dimethyl-4'-methoxydibenzoylmethan und 2,6-Dimethyl-4-*tert.*-butyl-4'-methoxydibenzoylmethan ausgewählt ist.

**26.** Verwendung nach Anspruch 25, dadurch gekennzeichnet, daß das Dibenzoylmethanderivat das 4,4'-Methoxy-*tert.*-butyldibenzoylmethan ist.

**27.** Verwendung nach Anspruch 25, dadurch gekennzeichnet, daß das Dibenzoylmethanderivat das 4-Isopropyldibenzoylmethanderivat ist.

**28.** Verwendung nach einem der Ansprüche 23 bis 27, dadurch gekennzeichnet, daß das Pigment vor der Behandlung mit einem Silicon mit einem Hüllstoff, der von einem Silicon verschieden ist, umhüllt ist.

**29.** Verwendung nach Anspruch 28, dadurch gekennzeichnet, daß der Hüllstoff unter Aluminiumoxid, Siliciumdioxid, Aluminiumverbindungen und Siliciumverbindungen oder den Gemischen dieser Verbindungen ausgewählt ist.

**30.** Verwendung nach einem der Ansprüche 23 bis 29, dadurch gekennzeichnet, daß die mittlere Größe der Elementarteilchen des Pigments im Bereich von 5 bis 100 nm liegt.

**31.** Verwendung nach Anspruch 30, dadurch gekennzeichnet, daß die Größe unter 50 nm liegt.

**32.** Verwendung nach einem der Ansprüche 23 bis 31, dadurch gekennzeichnet, daß das Silicon unter den Alkylsilanen, den Polydialkylsiloxanen und den Polyalkylhydrogenosiloxanen ausgewählt ist.

33. Verwendung nach Anspruch 32, dadurch gekennzeichnet, daß das Silicon unter Octyltrimethylsilan, den Polydimethylsiloxanen und den Polymethylhydrogenosiloxanen ausgewählt ist.

34. Verwendung nach einem der Ansprüche 23 bis 33, dadurch gekennzeichnet, daß das Pigment ein mit Octyltrimethylsilan behandeltes Titandioxid ist.

35. Verwendung nach einem der Ansprüche 23 bis 33, dadurch gekennzeichnet, daß das Pigment ein mit Aluminiumoxid umhülltes und mit einem Polydimethylsiloxan behandeltes Titandioxid ist.

36. Verwendung nach einem der Ansprüche 23 bis 33, dadurch gekennzeichnet, daß das Pigment ein mit einem Polydimethylsiloxan behandeltes Titandioxid ist.

37. Verwendung nach einem der Ansprüche 23 bis 33, dadurch gekennzeichnet, daß das Pigment ein mit einem Polymethylhydrogenosiloxan behandeltes Anatas/Rutil-Titandioxid ist.

38. Verwendung nach einem der Ansprüche 23 bis 33, dadurch gekennzeichnet, daß das Pigment ein mit Siliciumdioxid/Aluminiumoxid umhülltes und mit einem Silicon behandeltes Titandioxid ist.

39. Verwendung nach einem der Ansprüche 23 bis 38, dadurch gekennzeichnet, daß das Dibenzoylmethanderivat in der Zusammensetzung in einem Mengenanteil im Bereich von 0,1 bis 10 Gew.-%, bezogen auf das Gesamtgewicht der Zusammensetzung, vorliegt.

40. Verwendung nach Anspruch 39, dadurch gekennzeichnet, daß das Dibenzoylmethanderivat in der Zusammensetzung in einem Mengenanteil im Bereich von 0,3 bis 5 Gew.-%, bezogen auf das Gesamtgewicht der Zusammensetzung, vorliegt.

41. Verwendung nach einem der Ansprüche 23 bis 40, dadurch gekennzeichnet, daß das Pigment in der am Ende vorliegenden Zusammensetzung in einem Mengenanteil im Bereich von 0,1 bis 30 Gew.-%, bezogen auf das Gesamtgewicht der Zusammensetzung, vorliegt.

42. Verwendung nach Anspruch 41, dadurch gekennzeichnet, daß das Pigment in der am Ende vorliegenden Zusammensetzung in einem Mengenanteil im Bereich von 0,5 bis 20 Gew.-%, bezogen auf das Gesamtgewicht der Zusammensetzung, vorliegt.